# EUROPEAN PATENT APPLICATION

(11) **EP 2 130 927 A1**
(43) Date of publication of application: **09.12.2009**
(21) Application number: 08721361.7
(22) Date of filing: 05.03.2008
(51) Int. Cl.: C12Q 1/68, C12N 15/09

(54) **METHOD FOR AMPLIFICATION OF DNA FRAGMENT**

(30) Priority: 07.03.2007 JP 2007056839
(71) Applicant: The University of Tokyo, Bunkyo-Ku Tokyo 113-8654 (JP)
(72) Inventor: SHIOTA, Kunio, Tokyo 113-8654 (JP); YAGI, Shintaro, Tokyo 113-8654 (JP); TAKAHASHI, Yoko, Tokyo 113-8654 (JP); TANAKA, Satoshi, Tokyo 113-8654 (JP); OHGANE, Jun, Tokyo 113-8654 (JP)
(74) Representative: Woods, Geoffrey Corlett
(86) International application number: PCT/JP2008/053941
(87) International publication number: WO 2008/111453

(57) **Abstract**

To measure the methylation degree of a genomic DNA simply and correctly as well as exhaustively, there is provided a method for amplifying a DNA fragment, **characterized by** comprising the following steps (1) to (5):
(1) digesting a sample double-stranded DNA with a first restriction enzyme;
(2) adding a double-stranded DNA fragment consisting of a first oligonucleotide and a second oligonucleotide to a restriction enzyme cleavage end of the double-stranded DNA fragment produced in step (1) to obtain a DNA fragment;
(3) digesting the DNA fragment obtained in step (2) with a second restriction enzyme;
(4) allowing the double-stranded DNA fragment produced in step (3) to coexist with a third oligonucleotide and a fourth oligonucleotide in the presence of a ligase; and
(5) heating the double-stranded DNA fragment obtained in step (4), thereby dissociating the double-stranded DNA fragment and then performing a polymerase chain reaction to selectively amplify a DNA fragment having the cleavage end of the first restriction enzyme and the cleavage end of the second restriction enzyme.

SEQUENCE LISTING

<110> THE UNIVERSITY OF TOKYO

<120> METHOD FOR AMPLIFYING DNA FRAGMENTS

<130> N.108207

<140> EP 08721361.7
<141> 2008-03-05

<150> JP 2007/056839
<151> 2007-03-07

<160> 7

<170> PatentIn Ver. 2.1

<210> 1
<211> 24
<212> DNA
<213> Artificial Sequence

<220>
<223> Description of Artificial Sequence:synthetic DNA

<400> 1
aggcaactgt gctatccgag ggaa                                        24

<210> 2
<211> 12
<212> DNA
<213> Artificial Sequence

<220>
<223> Description of Artificial Sequence:synthetic DNA

<400> 2

ggccagcggt ga                                                     12

<210> 3
<211> 18
<212> DNA
<213> Artificial Sequence

<220>
<223> Description of Artificial Sequence:synthetic DNA

<400> 3
ggcaactgtg ctatccga                                               18

<210> 4

<211> 10
<212> DNA
<213> Artificial Sequence

<220>
<223> Description of Artificial Sequence:synthetic DNA

<400> 4
cgttccctcg                                                              10


<210> 5
<211> 24
<212> DNA
<213> Artificial Sequence

<220>
<223> Description of Artificial Sequence:synthetic DNA

<400> 5
agcactctcc agcctctcac cgct                                              24


<210> 6
<211> 10
<212> DNA
<213> Artificial Sequence

<220>
<223> Description of Artificial Sequence:synthetic DNA

<400> 6
cgagcggtga                                                              10


<210> 7
<211> 18
<212> DNA
<213> Artificial Sequence

<220>
<223> Description of Artificial Sequence:synthetic DNA

<400> 7
gcactctcca gcctctca                                                     18

## Description

### Technical Field

The present invention relates to a method for amplifying a DNA fragment and a method for measuring the degree of methylation of a genomic DNA.

### Background Art

Methylation which occurs at the 5th position of cytosine (C) in a CpG dinucleotide is the only modification observed in a genomic DNA of vertebrates including mammals and is strongly involved in regulation of gene activities. Specifically, chromatins are condensed by DNA methylation, resulting in inhibition of gene activities.

Genome-wide analyses of DNA methylation status in tissues and cells, which vary depending on the tissue-types and the development stages, with a focus on the CpG islands have revealed that a tissue-dependent differentially methylated region (T-DMR), a region showing a methylation status dependent to a tissue or a cell, exists in a genic region expressed in a tissue or a cell-type specific manner, and the methylation status of T-DMR is closely associated with regulation of tissue- or cell-specific gene expression. It is important for normal development and cell differentiation to establish and maintain a normal cell-type or tissue specific DNA methylation profile consisting of many patterns of the T-DMR methylation status in the whole genome (Shiota 2004, Cytogenet Genome Res, 105, 325-34).

Analyses of the methylation status in cancer suppressor genes using cancer cells or patient specimens have indicated that abnormal methylation should be one of major carcinogenesis mechanisms. Furthermore, it has been reported that abnormality in DNA methylation is not observed in genes associated with specific carcinogenesis alone, but, in cancer cells and cell groups in the stage before carcinogenesis, the overall methylation state of a genome DNA is in an abnormal modification state as hypomethylation. Furthermore, abnormal DNA methylation has been confirmed at an inflammation site in infection shown to be associated with carcinogenesis (Maekita, Nakazawa et al. 2006, Clin Cancer Res, 12, 989-95).

As described above, the DNA methylation profile of the whole genome differs between a normal cell and an abnormal cell, and normality of a cell or a tissue can be determined by identifying the methylation status of a genomic DNA. For example, prediagnosis and diagnosis of diseases such as cancer can be made by determining the methylation state of a genomic DNA (Ushijima 2005, Nat Rev Cancer, 5, 223-31). Furthermore, since a DNA methylation profile consisting of methylation status of numerous T-DMRs over the whole genome is cell-type or tissue-specific, identification of a cell-type or a tissue, differentiation of normal and abnormal cells or tissues, and diagnosis of the presence or absence of a disease can be achieved by using the DNA methylation profile. Furthermore, a T-DMR which is important for diagnosis or identification can be identified.

Methods for investigating the DNA methylation status are classified into methods using selective cleavages with methylation-sensitive restriction enzymes, methods comprising screening for a methylated DNA itself using an antibody or a methylated DNA-binding protein, and methods utilizing a bisulfite reaction.

Examples of methods for obtaining a genome-wide DNA methylation profile includes a restriction landmark genomic scanning (RLGS), which is an analysis using a methylation-sensitive restriction enzyme. In the RLGS, a DNA methylation profile can be visualized as an image composed of many two-dimensional spots by labeling cleavage sites of NotI, a methylation-sensitive restriction enzyme, with a radioactive isotope and performing the cleavage with a non-methylation-sensitive restriction enzyme and two-dimensional electrophoresis in combination (Ohgane, Aikawa et al., 1998, Dev Genet, 22, 132-40). Furthermore, by employing a viRLGS, which simulates the RLGS based on genome database information, in combination, genetic information of spots constituting a DNA methylation profile can also be predicted (Hattori, Abe et al. 2004, Genome Res, 14, 1733-40). To determine whether the predicted genes actually correspond to the spots constituting the profile, however, more detailed analyses are required. In other words, the RLGS is a method suitable for obtaining a genome-wide DNA methylation profile, but the T-DMR identification process is difficult.

Information about recognition sites of specific restriction enzymes can be obtained based on the genome information, and whether these individual regions are methylated can be investigated. In this case, the methylation-sensitive (MS)-PCR method can be used. In the MS-PCR method, PCR primers are designated so that the methylation-sensitive restriction enzyme recognition sites should be flanked, and a fragment treated with a methylation-sensitive restriction enzyme and an untreated fragment are subjected to PCR. Since a fragment is not cleaved when the recognition site of the methylation-sensitive restriction enzyme is methylated, DNA is amplified by PCR. However, since a fragment is cleaved when it is not methylated, amplification by PCR does not occur. Since the amount of the fragments amplified by PCR reflects the amount of non-methylated DNA, the methylation status of a target site can be estimated by quantitating the amount of PCR-amplified fragments. A genome-wide DNA methylation profile can be efficiently obtained by primers at more than one site, when those site have been identified as T-DMRs. Comprehensive analyses of the whole genome based on MS-PCR are enabled by using PCR primer sets established at thousands or tens of thousands of restriction sites (WO2007/088744). However, since which site should be analyzed to identify a new unknown T-DMR is not known, thousands or tens of thousands of reactions need to be performed, which is inefficient.

As comprehensive analysis methods, methods using a microarray have been reported. In the method using a microarray, adjustment of the number of probes to be mounted on the array and fragments to be hybridized is an important factor for determining exhaustiveness. As an analysis method using a methylation-sensitive restriction enzyme, a method of comparing the amounts of fragments produced by methylation-sensitive and non-methylation-sensitive restriction enzymes has been reported. For example, in HpaII tiny fragment enrichment by ligation mediated PCR (HELP) method, fragments treated with a methylation-sensitive enzyme (*Hpa*II) and a non-methylation-sensitive enzyme (*Msp*I) that have an identical recognition sequence are amplified by PCR, and *Hpa*II fragments and *Msp*I fragments produced from the same DNA sample are competitively hybridized with a probe on a microarray to measure the ratio of the amounts of these fragments. This value expresses the methylation status of a specific restriction enzyme recognition sequence (Non-Patent Document 1). Similarly, in Microarray-based integrated analysis of methylation by isoschizomers (MIAMI) method, *Msp*I fragments or *Hpa*II fragments from different samples are competitively hybridized with a probe on a microarray to compare the methylation status of a specific restriction enzyme recognition sequence between different samples (Non-Patent Document 2). To amplify *Hpa*II- and *Msp*I-digested fragments, a ligation-mediated PCR (LM-PCR) method is used in which these fragments are ligated to an adaptor that can be ligated to the cleavage sites and amplified by PCR using a primer specific to the adaptor sequences.

Furthermore, a microarray analysis method is also available utilizing a principle that, when fragments are digested with a non-methylation-sensitive restriction enzyme, then bound with an adaptor, and treated with a methylation-sensitive restriction enzyme, methylated fragments are is amplified, but non-methylated fragments cannot be amplified (same principle as that of MS-PCR). Furthermore, a method, which is claimed to have improved accuracy, is also available by further combining enzyme cleavages intricately.
Non-Patent Document 1: Khulan, Thompson et al. 2006, Genome Res, 16, 1046-55
Non-Patent Document 2: Hatada, Fukasawa et al. 2006, Oncogene, 25, 3059-64

### Disclosure of the Invention

### Problems to be Solved by the Invention

In general, 2-kb or shorter fragments are efficiently replicated by PCR amplification, and 5-kb or longer fragments are not suitable for amplification.
Such a bias in PCR needs to be taken into account in amplification of the above-mentioned non-methylated fragments cleaved with a methylation-sensitive enzyme.

The recognition sequence of *Hpa*II, a methylation-sensitive enzyme, is four base pairs CCGG. When it is assumed that a genomic DNA, in which the four nucleotides are uniformly distributed, is completely digested, fragments of average 265 base pairs are expected. Due to biased distribution of nucleotide sequences are on a mammal genome, however, the frequency of CpG with observed methylation is approx. 1/5 of the expected frequency. Therefore, the frequency of *Hpa*II fragments including CpG is also low. In fact, when an analysis is performed based on the nucleotide sequence of mouse chromosome 17, the average length of *Hpa*II fragments is approx. 1.4 kb, which is longer than when each nucleotide is assumed to be uniformly distributed. Due to methodological restrictions of PCR and the positional bias of recognition sites of restriction enzymes, the following problems arise in analyses using a single recognition site.

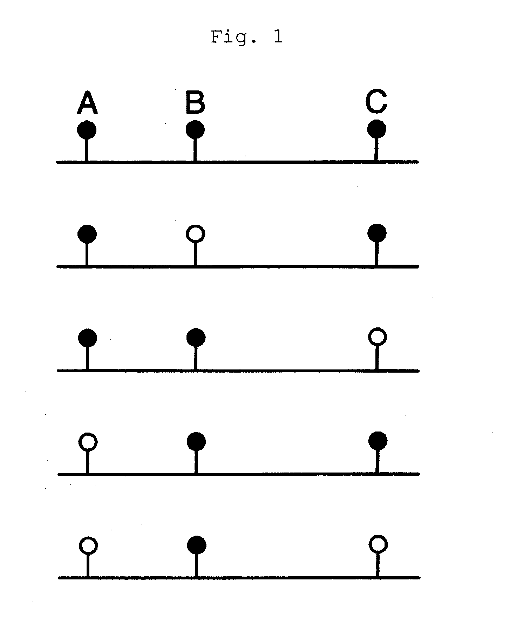

For example, when an analysis is performed using cleavage of a fragment with *Hpa*II as an indicator, three *Hpa*II sites A, B, and C shown in Figure 1 occur. A and B and B and C have a distance therebetween with which a fragment can be efficiently amplified by PCR. However, when the distance between A and C is too long to be efficiently amplified by PCR, the amplification of fragment between A and C does not occur, and no signal can be obtained in any combination of methylation states shown in Figure 1. That is, methylated *Hpa*II recognition sites cannot be identified in this case. When a methylation-sensitive enzyme which has a lower frequency of recognition sites than that of *Hpa*II, for example, *Sma*I or *Not*I, is used, it is apparent from the following description that the methylation status of each DNA cannot be determined by a standard LM-PCR method.

In other words, when a restriction enzyme with a long recognition sequence is used, most fragments are too long to be amplified by PCR. For example, *Not*I used in the RLGS recognizes and cleaves an 8-bp sequence, GCGGCCGC. Since the average chain length of the fragment expected from the mouse genome sequence is 371 kb, most fragments cannot be amplified by PCR (details are described in Reference Example). The *Sma*I recognition sequence is 7 kb in length, and most fragments are not to be analyzed when the LM-PCR method is used. Furthermore, since the occurrence of amplification of a fragment depends on the methylation state of one of the two ends as shown using *Hpa*II as an example, the analysis will become less comprehensive.

As described above, since a digested fragment is not amplified by LM-PCR when the distance between cleavage sites of a methylation-sensitive restriction enzyme is long, there is a problem that the methylation status of a cleavage site cannot be determined. Against such a technical background, an object of the present invention is to provide means with which the occurrence of a cleaved fragment can be correctly determined even when the distance between cleavage sites of a methylation-sensitive restriction enzyme is long.

### Means for Solving the Problems

The inventors of the present invention conducted various researches to achieve the foregoing object. As a result, they found that the methylation status of a cleavage site can be correctly determined even when the distance between cleavage sites of a methylation-sensitive restriction enzyme is long, by treating a genomic DNA with a methylation-sensitive restriction enzyme and then treating with a non-methylation-sensitive restriction enzyme to selectively amplify only a fragment having cleavage ends of the methylation-sensitive restriction enzyme and the non-methylation-sensitive restriction enzyme, and accomplished the present invention.

Specifically, the present invention provides the following [1] to [8].
[1] A method for amplifying a DNA fragment, characterized by comprising the following steps (1) to (5):
   (1) digesting a sample double-stranded DNA with a first restriction enzyme;
   (2) adding a double-stranded DNA fragment consisting of a first oligonucleotide and a second oligonucleotide having a sequence complementary to the first oligonucleotide to a restriction enzyme cleavage end of the double-stranded DNA fragment produced in step (1) to obtain a DNA fragment, in which the 5' end of the first oligonucleotide and the 3' end of the second oligonucleotide are ligated to the restriction enzyme cleavage end of the double-stranded DNA fragment produced in step (1);
   (3) digesting the DNA fragment obtained in step (2) with a second restriction enzyme;
   (4) allowing the double-stranded DNA fragment produced in step (3) to coexist with a third oligonucleotide with an unphosphorylated 5' end and a fourth oligonucleotide with an unphosphorylated 5' end that has a sequence complementary to the third oligonucleotide in the presence of a ligase, thereby adding a double-stranded DNA fragment consisting of the third oligonucleotide and the fourth oligonucleotide to the second restriction enzyme cleavage end of the double-stranded DNA fragment produced in step (3) to obtain a DNA fragment, in which the 3' end of the fourth oligonucleotide is ligated to the restriction enzyme cleavage end of the double-stranded DNA fragment produced in step (3), but the 5' end of the third oligonucleotide is not ligated to the restriction enzyme cleavage end of the double-stranded DNA fragment produced in step (3); and
   (5) heating the double-stranded DNA fragment obtained in step (4) thereby dissociating the double-stranded DNA fragment, and then performing a polymerase chain reaction using an oligonucleotide hybridizable with the first oligonucleotide and an oligonucleotide hybridizable with the third oligonucleotide as primers to selectively amplify a DNA fragment having the cleavage end of the first restriction enzyme and the cleavage end of the second restriction enzyme.
[2] The method for amplifying a DNA fragment according to [1], **characterized in that** the step (2) is allowing the double-stranded DNA fragment produced in step (1) to coexist with the first oligonucleotide with an unphosphorylated 5' end and the second oligonucleotide with an unphosphorylated 5' end in the presence of a ligase, threreby adding a double-stranded DNA fragment consisting of the first oligonucleotide and the second oligonucleotide to the restriction enzyme cleavage end of the double-stranded DNA fragment produced in step (1) to obtain a DNA fragment, in which the 3' end of the second oligonucleotide is ligated to the restriction enzyme cleavage end of the double-stranded DNA fragment produced in step (1), but the 5' end of the first oligonucleotide is not ligated to the restriction enzyme cleavage end of the double-stranded DNA fragment produced in step (1), and then allowing a DNA polymerase to act to ligate the double-stranded DNA fragment produced in step (1) and the 5' end of the first oligonucleotide.
[3] The method for amplifying a DNA fragment according to [1], **characterized in that** the step (2) is allowing the double-stranded DNA fragment produced in step (1) to coexist with the first oligonucleotide with a phosphorylated 5' end and the second oligonucleotide with a phosphorylated 5' end in the presence of a ligase, threreby adding a double-stranded DNA fragment consisting of the first oligonucleotide and the second oligonucleotide to the restriction enzyme cleavage end of the double-stranded DNA fragment produced in step (1) to obtain a DNA fragment, in which the 5' end of the first oligonucleotide and the 3' end of the second oligonucleotide are ligated to the restriction enzyme cleavage end of the double-stranded DNA fragment produced in step (1), and then removing the first oligonucleotide, the second oligonucleotide, and the DNA fragment produced by ligation.
[4] The method for amplifying a DNA fragment according to any of [1] to [3], **characterized in that** the first restriction enzyme and the second restriction enzyme are restriction enzymes which leave a 5' overhang, and the first oligonucleotide and the third oligonucleotide are oligonucleotides having a sequences complementary to the 5' overhangs generated by the first restriction enzyme and the second restriction enzyme, respectively.
[5] A method for measuring the methylation levels of a genomic DNA by allowing a methylation-sensitive restriction enzyme to act on the genomic DNA, amplifying the resulting DNA fragment, and determining the methylation degree of a cleavage site of the methylation-sensitive restriction enzyme based on the amplification degree, characterized by comprising the following steps (1) to (6):
   (1) digesting a genomic DNA with a methylation-sensitive restriction enzyme;
   (2) adding a double-stranded DNA fragment consisting of a first oligonucleotide and a second oligonucleotide having a sequence complementary to the first oligonucleotide to a restriction enzyme cleavage end of a DNA fragment produced in step (1) to obtain a DNA fragment, in which the 5' end of the first oligonucleotide and the 3' end of the second oligonucleotide are ligated to the restriction enzyme cleavage end of the DNA fragment produced in step (1);
   (3) digesting the DNA fragment obtained in step (2) with a non-methylation-sensitive restriction enzyme;
   (4) allowing the double-stranded DNA fragment produced in step (3) to coexist with a third oligonucleotide with an unphosphorylated 5' end and a fourth oligonucleotide with an unphosphorylated 5' end that has a sequence complementary to the third oligonucleotide in the presence of a ligase, threreby adding a double-stranded DNA fragment consisting of the third oligonucleotide and the fourth oligonucleotide to a non-methylation-sensitive restriction enzyme cleavage end of the double-stranded DNA fragment produced in step (3) to obtain a DNA fragment, in which the 3' end of the fourth oligonucleotide is ligated to the restriction enzyme cleavage end of the double-stranded DNA fragment produced in step (3), but the 5' end of the third oligonucleotide is not ligated to the restriction enzyme cleavage end of the double-stranded DNA fragment produced in step (3);
   (5) heating the double-stranded DNA fragment obtained in step (4) thereby dissociating the double-stranded DNA fragment, and then performing a polymerase chain reaction using an oligonucleotide hybridizable with the first oligonucleotide and an oligonucleotide hybridizable with the third oligonucleotide as primers to selectively amplify a DNA fragment having a cleavage end of a methylation-sensitive restriction enzyme and a cleavage end of a non-methylation-sensitive restriction enzyme; and
   (6) identifying the position of the amplified DNA fragment on the genomic DNA and determining the amplification amount of a DNA fragment including a cleavage site for each methylation-sensitive restriction enzyme.
[6] The method for measuring the methylation degree of a genomic DNA according to [5], **characterized in that** the step (2) is allowing the double-stranded DNA fragment produced in step (1) to coexist with a first oligonucleotide with an unphosphorylated 5' end and a second oligonucleotide with an unphosphorylated 5' end in the presence of a ligase, and adding a double-stranded DNA fragment consisting of the third oligonucleotide and the fourth oligonucleotide to a restriction enzyme cleavage end of the double-stranded DNA fragment produced in step (1) to obtain a DNA fragment, in which the 3' end of the second oligonucleotide is ligated to the restriction enzyme cleavage end of the double-stranded DNA fragment produced in step (1), but the 5' end of the first oligonucleotide is not ligated to the restriction enzyme cleavage end of the double-stranded DNA fragment produced in step (1), and then allowing a DNA polymerase to act to ligate the double-stranded DNA fragment produced in step (1) and the 5' end of the first oligonucleotide.
[7] The method for measuring the methylation degree of a genomic DNA according to [5], **characterized in that** the step (2) is allowing the double-stranded DNA fragment produced in step (1) to coexist with a first oligonucleotide with a phosphorylated 5' end and a second oligonucleotide with a phosphorylated 5' end in the presence of a ligase, threreby adding a double-stranded DNA fragment consisting of the first oligonucleotide and the second oligonucleotide to a restriction enzyme cleavage end of the double-stranded DNA fragment produced in step (1) to obtain a DNA fragment, in which the 5' end of the first oligonucleotide and the 3' end of the second oligonucleotide are ligated to the restriction enzyme cleavage end of the double-stranded DNA fragment produced in step (1), and removing the first oligonucleotide, the second oligonucleotide, and the DNA fragment produced by ligation.
[8] The method for measuring the methylation degree of a genomic DNA according to any one of [5] to [7], **characterized in that** the methylation-sensitive restriction enzyme and the non-methylation-sensitive restriction enzyme are restriction enzymes that leave a 5' overhang, and the first oligonucleotide and the third oligonucleotide are oligonucleotides having sequences complementary to the 5' overhangs generated by the methylation-sensitive restriction enzyme and the non-methylation-sensitive restriction enzyme, respectively. Advantage of the Invention

According to the method for amplifying a DNA fragment of the present invention, whether a DNA has been cleaved with a restriction enzyme can be correctly determined. By using this amplification method for an analysis of the methylation status of a genomic DNA, the methylation levels of the genomic DNA can be measured simply and correctly as well as comprehensively. Furthermore, the amplification method of the present invention can also be utilized in not only analyses of methylation status but also analyses of single nucleotide polymorphism.

### Brief Description of the Drawings

Figure 1 is a schematic diagram of a region that is hardly amplified by LM-PCR. In the diagram, o denotes a unmethylated state (cleaved with *Hpa*II), and • denotes a methylated state (not cleaved with *Hpa*II). Of three *Hpa*II sites, A, B, and C, the distances between A and B and B and C can produce fragments that can be efficiently amplified by PCR, but when the distance between A and C is long, and a fragment is not efficiently amplified, the A-C fragment is not amplified in any cases shown. Therefore, whether cleavage has occurred or not cannot be determined by the occurrence of amplification of a fragment;

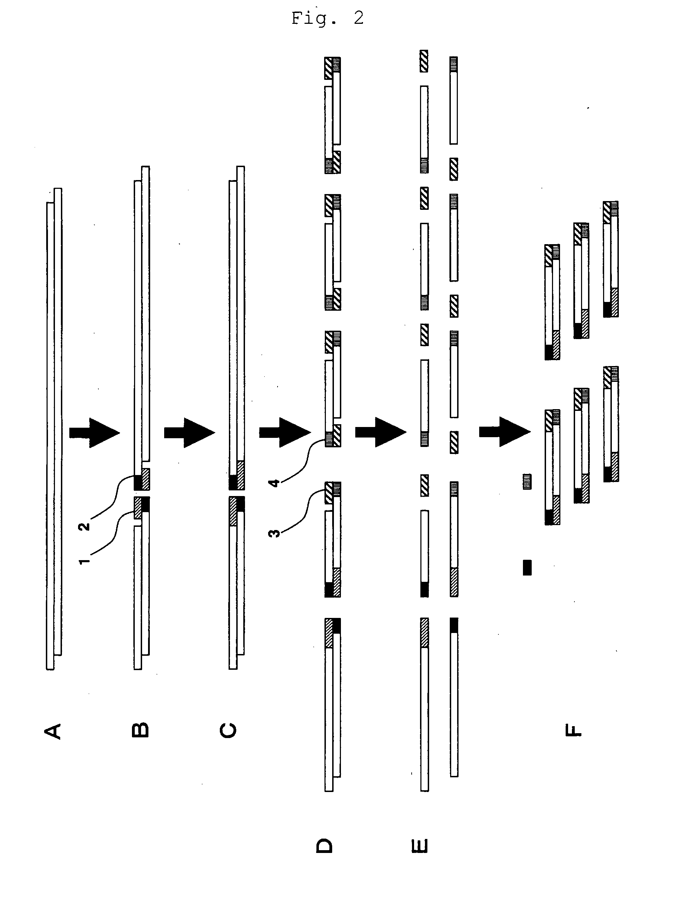

Figure 2 is a schematic diagram illustrating the method for amplifying a DNA fragment of the present invention;

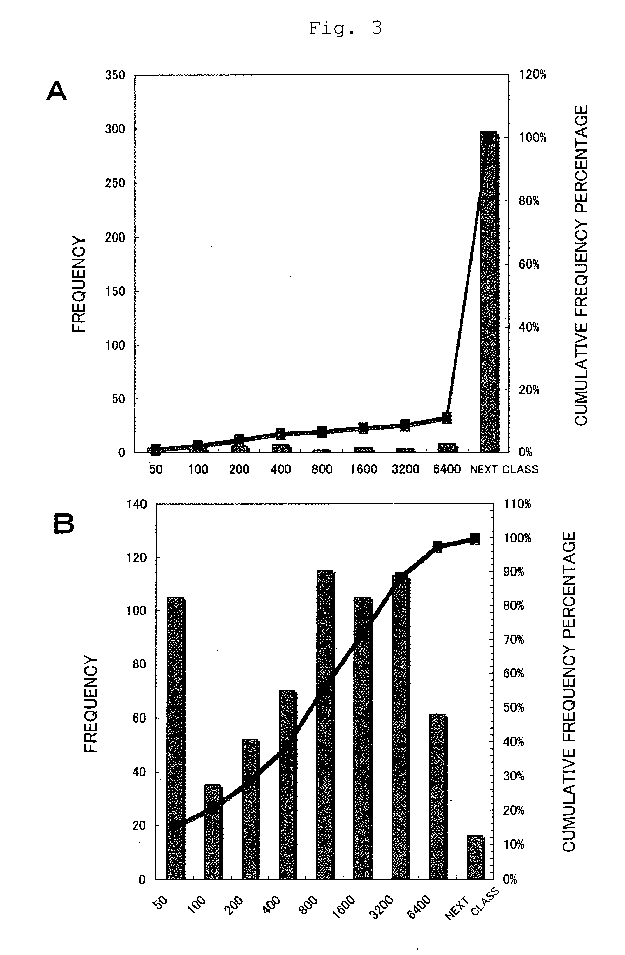

Figure 3 illustrates analysis results of the length of a fragment produced after digestion of an completely unmethylated mouse genomic DNA with *Not*I. Figure 3A is a histogram showing a distribution of lengths of fragments produced when mouse chromosome 17 was digested with *Not*I. Bars represent frequencies, and lines represent cumulative frequency percentages, with intervals shown below. It is shown that, when the DNA was digested with *Not*I alone, most fragments exceeded 6.4 kb. Figure 3B is a histogram showing a distribution of lengths of fragments produced when mouse chromosome 1 was digested with *Not*I and *Taq*I. After digestion with *Taq*I, most fragments were distributed at 3.2 kb or below;

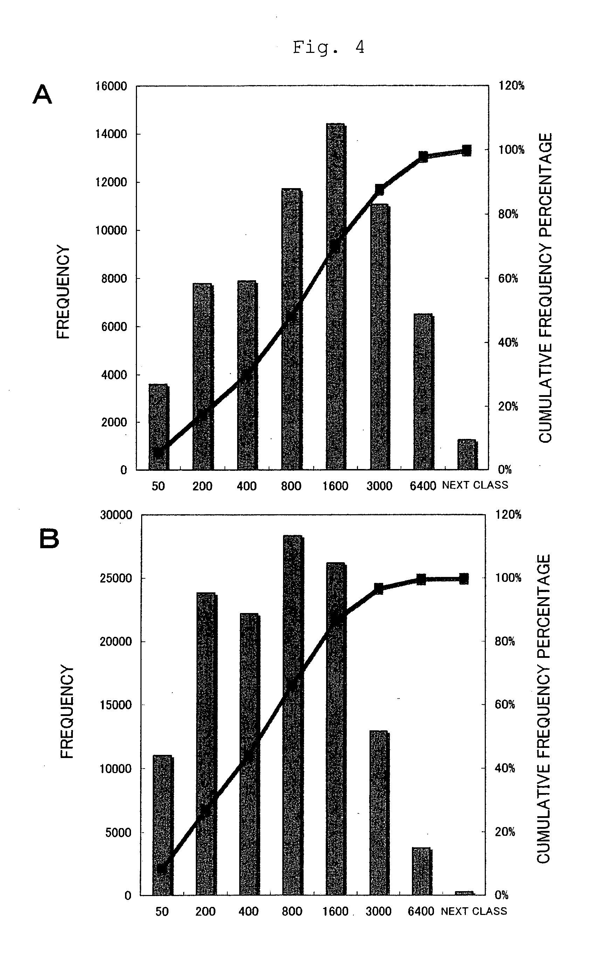

Figure 4 illustrates analysis results of lengths of fragments produced after digestion of an completely unmethylated mouse genomic DNA with *HpyCH4*IV. Figure 4A is a histogram showing a distribution of lengths of fragments produced when mouse chromosome 17 was digested with *HpyCH4*IV. Bars represent frequencies, and lines represent cumulative frequency percentages, with intervals shown below. Figure 4B is a histogram showing a distribution of lengths of fragments produced when mouse chromosome 17 was digested with *HpyCH4*IV and *Taq*I;

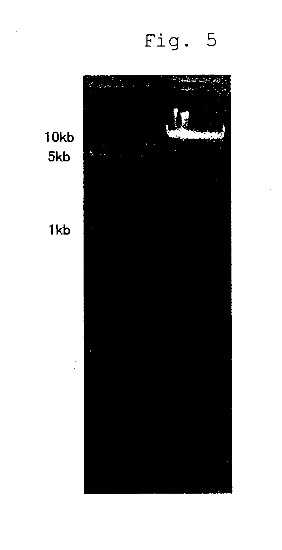

Figure 5 illustrates results of an agarose electrophoresis of a mouse ES cell genomic DNA digested with *HpyCH4*IV. The leftmost lane is a molecular weight marker, and the positions of 1-, 5-, and 10-kb fragments are shown on the left of the figure. The electrophoresis pattern of a mouse ES cell genome digested with *HpyCH4*IV is shown. As shown in Figure 4, when a mouse genome in a completely unmethylated status is digested with *HpyCH4*IV, fragments having an average molecular weight of 1.4 kb are produced, but these are rarely detected from the electrophoretic profile. Therefore, it is found that most *HpyCH4*IV sites of a mouse genome are methylated, and therefore few fragments have lengths that are easily amplified by PCR when the DNA is cleaved with *HpyCH4*IV alone;

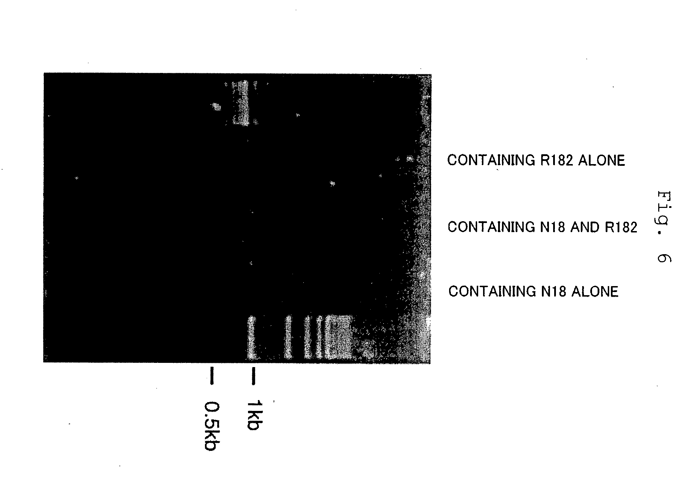

Figure 6 illustrates agarose electrophoresis confirmation results of selective PCR amplification of a fragment having the *Not*I cleavage end. From left of the figure, a low molecular weight marker, a part of the PCR reaction mixture containing R182 alone as a primer, a part of the PCR reaction mixture containing N18 and R182, a part of the PCR reaction mixture containing N18 alone, and a high molecular weight marker were subjected to electrophoresis;

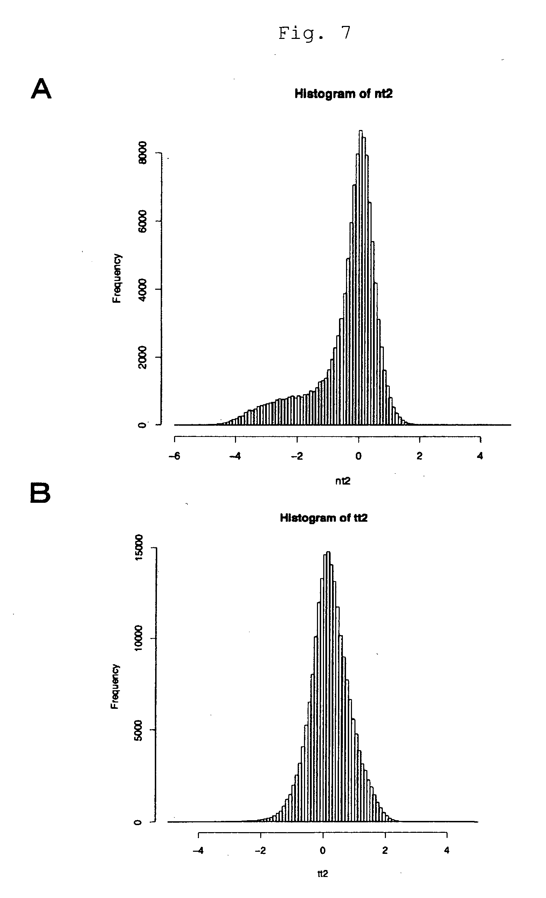

Figure 7 illustrates microarray confirmation results of selective amplification of a fragment having the NotI cleavage end. Probes on a microarray were divided into the *Not*I-*Taq*I group and the *Taq*I-*Taq*I group, and distributions of logarithmic values of fluorescence intensity ratios of Cy3 and Cy5 of probes belonging to each group are shown in a histogram (*Not*I-*Taq*I group, Figure 7A; *Taq*I-*Taq*I group, Figure 7B). These figures demonstrate that fragments having the *Not*I site at an end were selectively amplified;

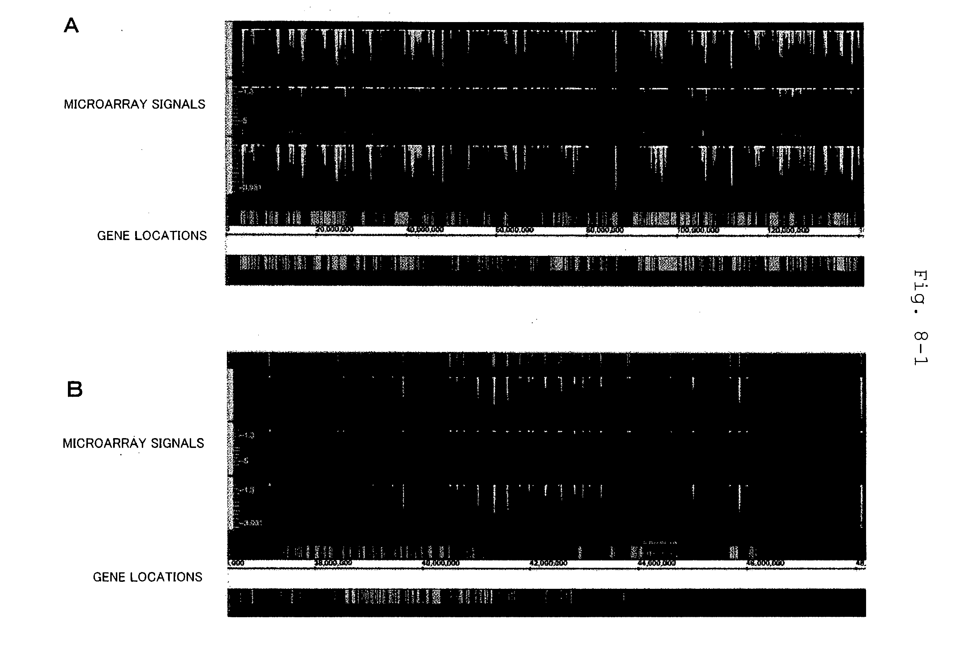

Figure 8-1 illustrates IGB (Integrated Genome Browser) analysis results of chromosome 7 of a mouse liver genomic DNA. Figure 8-1A illustrates hybridization signals of chromosome 7 of the mouse liver genomic DNA. The top column illustrates signals of all probes, the middle column illustrates signals of probes belonging to the *Not*I-*Taq*I group. The lower column illustrates signals of probes belonging to the *Taq*I*-Taq*I group. Figure 8-1B is an enlarged view of a part of Figure 8-1A, and *Not*I cleavage sites are shown above signals of all probes;

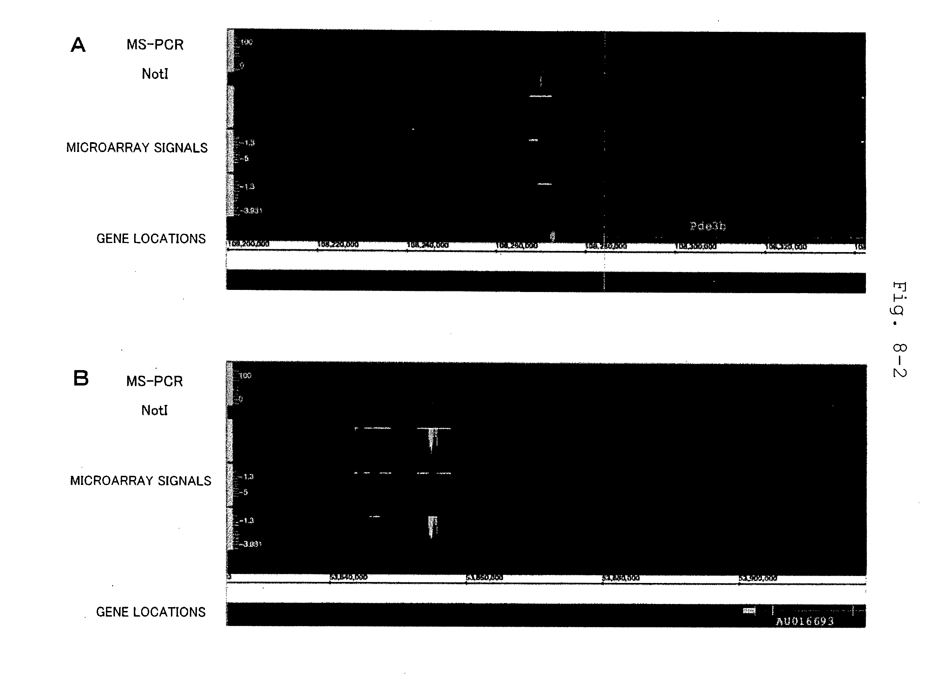

Figure 8-2 illustrates IGB analysis results of chromosome 7 of the mouse liver genomic DNA. Figure 8-2A shows hybridization signals of regions including *Not*I cleavage sites which are found by MS-PCR to be methylated. Figure 8-2B shows hybridization signals of regions including *Not*I cleavage sites which are found by MS-PCR to be non-methylated. The top column shows methylation states (sites at which downward signals are detected indicate that fragments have been amplified and are in a hypomethylation state). The second column from the top shows *Not*I cleavage sites. The third column from the top shows signals of all probes. The fourth column from the top shows signals of probes belonging to the *Not*I*-Taq*I group. The fifth column from the top shows signals of probes belonging to the *Taq*I-*Taq*I group;

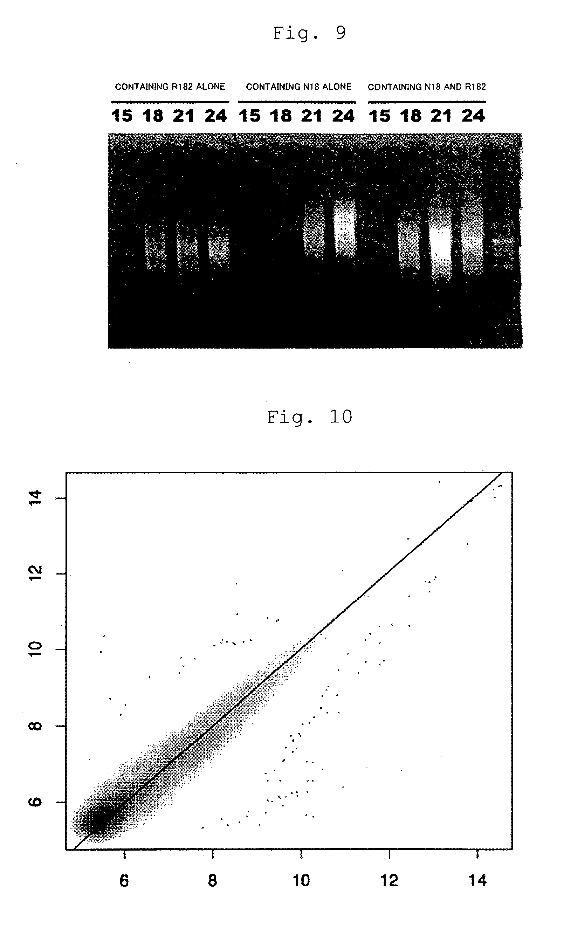

Figure 9 illustrates agarose electrophoresis confirmation results of selective PCR amplification of fragments having the *Not*I cleavage end. From the left of the figure, a part of a PCR reaction mixture containing R182 alone as a primer (from left, electrophoresis of products after 15, 18, 21, and 24 cycles), a part of a PCR reaction mixture containing N18 alone as a primer (from left, electrophoresis of products after 15, 18, 21, and 24 cycles), a part of a PCR reaction mixture containing N18 and R182 alone as primers (from left, electrophoresis of products after 15, 18, 21, and 24 cycles), and a high molecular weight marker were subjected to electrophoresis;
Figure 10 is a correlation plot of microarray signals for confirming reproducibility. The PCR amplification product in Example 5 was hybridized with probes on a microarray, and signal intensity of each probe was measured. This procedure was repeated twice, and two measured values were plotted on the vertical axis and the horizontal axis for each probe;

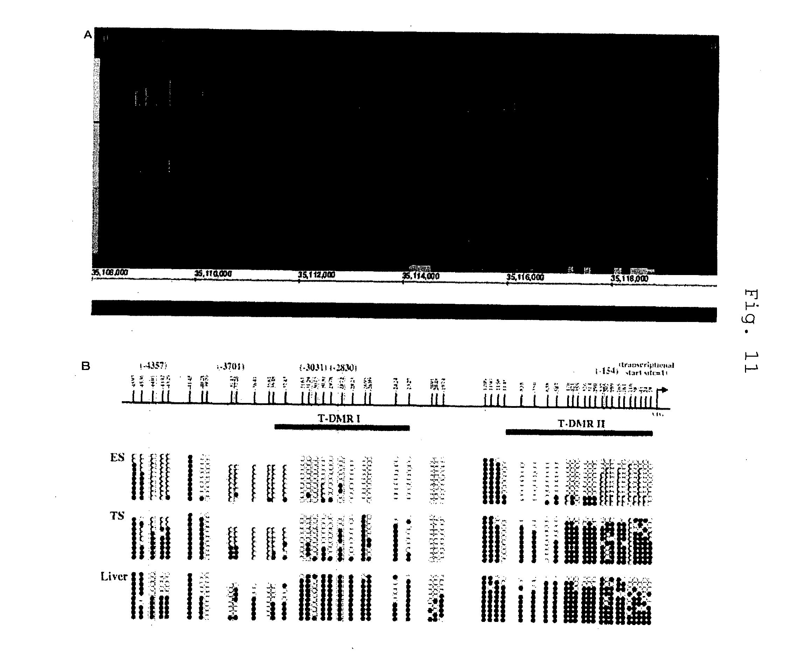

Figure 11 illustrates microarray analysis results of chromosome 17. Figure 11A shows a microarray analysis example of the *Oct4* (*Pou5fi1*) gene region located on chromosome 17. From the top, *Taq*I cleavage sites, *HpyCH4*IV cleavage sites, ES cell profiles, TS cell profiles, and liver profiles are shown. The upward bar denotes the MAT score of each probe, and detection of an upward signal means that the fragment in this region has been amplified. Furthermore, this signal correlates to the methylation state of the *HpyCH4*IV site in the amplified region. A high value means that the cleavage site is in a more hypomethylated state. Figure 11B shows bisulfite sequencing results of the regions shown in Figure 11A. The • in the figure denotes that cytosine at this site is methylated, and the o denotes cytosine is not methylated. Furthermore, the site surrounded by a broken line indicates a *HpyCH4*IV cleavage site; and

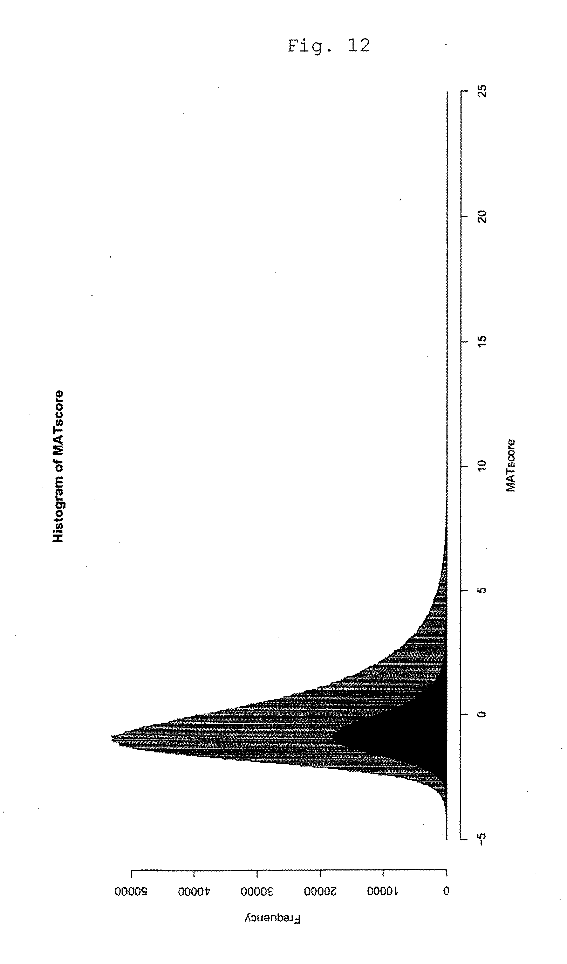

Figure 12 is a histogram showing a distribution of MATscores of probes that exist in fragments including *HpyCH4*IV cleavage sites and other probes. The grey histogram is for probes that exist in fragments including *HpyCH4*IV cleavage sites, and the black histogram is for other probes.

### Description of Symbols

- 1: First oligonucleotide
- 2: Second oligonucleotide
- 3: Third oligonucleotide
- 4: Fourth oligonucleotide

### Best Mode for Carrying Out the Invention

Hereafter, the present invention will be explained in detail.

First, the principle of the method for amplifying a DNA fragment of the present invention will be explained with reference to Figure 2.

A genomic DNA (Figure 2A) is digested with a first restriction enzyme, and the cleaved fragment is allowed to coexist with a first oligonucleotide 1 and a second oligonucleotide 2 in the presence of a ligase. Since the 5' end of the first oligonucleotide 1 is not phosphorylated, it is not ligated to the 3' end of the cleaved fragment (Figure 2B). On the other hand, since the second oligonucleotide 2 is a sequence complementary to the first oligonucleotide 1, it is hybridized with the first oligonucleotide 1 and ligated to the 3' end of the cleaved fragment by an action of a ligase (Figure 2B).
Two kinds of oligonucleotides are thus added to the cleaved fragment produced by the first restriction enzyme, and a DNA polymerase is allowed to act to ligate the 5' end of the first oligonucleotide 1 and the 3' end of the cleaved fragment (Figure 2C). When an oligonucleotide with an already phosphorylated 5' end is used, such a procedure using a DNA polymerase is unnecessary. In this case, however, the ligated oligonucleotides become targets of each other in subsequent PCR, and a PCR reaction may be competitively inhibited. Therefore, it is preferable to remove such an oligonucleotide.

After the above procedures, the DNA fragment is cleaved with a second restriction enzyme, and the cleaved fragment is allowed to coexist with a third oligonucleotide 3 and a fourth oligonucleotide 4 in the presence of a ligase. Since the 5' end of the third oligonucleotide 3 is not phosphorylated as with the first oligonucleotide 1, this end is not ligated to the 3' end of a fragment cleaved with the second restriction enzyme (Figure 2D). Since the fourth oligonucleotide 4 has a sequence complementary to the third oligonucleotide 3 as with the second oligonucleotide 2, it is hybridized with the third oligonucleotide 3 and ligated to the 3' end of the cleaved fragment by an action of the ligase (Figure 2D). Two kinds of oligonucleotides are thus added to the cleaved fragment produced by the second restriction enzyme, then the double-stranded DNA fragment is heated to dissociate it to single strands. The third oligonucleotide 3 is dissociated from the genomic DNA fragment by this heat treatment. After this heat treatment, PCR is performed using the second oligonucleotide 2 and the fourth oligonucleotide 4 as primers. The second oligonucleotide 2 binds to the first oligonucleotide 1 portion of the single-stranded DNA fragment, and the DNA strand is elongated. However, since the fourth oligonucleotide 4 binds to the third oligonucleotide 3 dissociated from the DNA fragment, the DNA strand is not elongated (Figure 2E). The fourth oligonucleotide 4 also binds to the DNA strand elongated from the second oligonucleotide 2 and, in this case, elongates a DNA strand. As a result, a DNA fragment having the cleavage end of the second restriction enzyme at either ends is not amplified, and a DNA fragment having the cleavage end of the first restriction enzyme at one end and the cleavage end of the second restriction enzyme at the other end is selectively amplified (Figure 2F).

Each step of the method for amplifying a DNA fragment of the present invention will be explained below.
Step (1) is digesting a sample double-stranded DNA with the first restriction enzyme. The first restriction enzyme is not limited, but, since the method for amplifying a DNA fragment of the present invention is mainly used in the analysis of the methylation state of a genomic DNA, methylation-sensitive restriction enzymes are preferred. Examples of the methylation-sensitive restriction enzyme include *Aat*II, *Acl*I, *Age*I, *Asc*I, *Ava*I, *BmgB*I, *BsiW*I, *BspD*I, *BstB*I, *Cla*I, *Eag*I, *Fse*I, *Fsp*I, *Hae*II, *Hha*I, *Hpy99*I, *HpyCH4*IV, *Hpa*II, *Kas*I, *Mlu*I, *Nar*I, *Nae*I, *Not*I, *Nru*I, *Pml*I, *Pvu*I, *Rsr*II, *Sac*II, *Sal*I, *SfoI,* SgrAi, *Sma*I, *Sna*BI, and so forth. Since the method for amplifying a DNA fragment of the present invention can also be used for analyses of single nucleotide polymorphism or the like, a restriction enzyme that cleaves only a specific genotype DNA or the like may be used as the first restriction enzyme. Furthermore, the first restriction enzyme may produce any of a blunt-end, a 5' overhang, and a 3' overhang. The sample double-stranded DNA is not particularly limited, and a genomic DNA or a DNA of a gene having polymorphism can be used as a sample. A problem may occur in procedures such as DNA extraction when a DNA fragment produced by the first restriction enzyme is very long. Therefore, in this case, a sample double-stranded DNA may be cleaved with another restriction enzyme other than the first restriction enzyme and the second restriction enzyme.
Step (2) is adding a double-stranded DNA fragment consisting of the first oligonucleotide and the second oligonucleotide having a sequence complementary to the first oligonucleotide to the restriction enzyme cleavage end of the double-stranded DNA fragment produced in step (1) to obtain a DNA fragment, in which the 5' end of the first oligonucleotide and the 3' end of the second oligonucleotide are ligated to the restriction enzyme cleavage end of the double-stranded DNA fragment produced in step (1). The first oligonucleotide and the second oligonucleotide may be similar to oligonucleotides referred to as adaptors in usual LM-PCR. The length of the first oligonucleotide is not particularly limited and is preferably 6 to 50 nucleotides. The length of the second oligonucleotide is not particularly limited either and is preferably 6 to 50 nucleotides. In order to add a double-stranded DNA fragment consisting of the first oligonucleotide and the second oligonucleotide to the double-stranded DNA fragment produced in step (1), it is necessary that the double-stranded DNA fragment produced in step (1) is allowed to coexist with the first oligonucleotide with an unphosphorylated 5' end and the second oligonucleotide with an unphosphorylated 5' end in the presence of a ligase, threreby adding the double-stranded DNA fragment consisting of the first oligonucleotide and the second oligonucleotide to the restriction enzyme cleavage end of the double-stranded DNA fragment produced in step (1) to obtain a DNA fragment, in which the 3' end of the second oligonucleotide is ligated to the restriction enzyme cleavage end of the double-stranded DNA fragment produced in step (1), but the 5' end of the first oligonucleotide is not ligated to the restriction enzyme cleavage end of the double-stranded DNA fragment produced in step (1), and then a DNA polymerase is allowed to act to ligate the double-stranded DNA fragment produced in step (1) and the 5' end of the first oligonucleotide. It is sufficient to use a ligase used in usual LM-PCR, and T4 ligase, *E. coli* DNA ligase, Taq DNA1 ligase, or the like can be used, for example. As a DNA polymerase, a DNA polymerase I Klenow fragment can be used. Enzymes other than Klenow fragments may be used as a DNA polymerase, but enzymes with a weak 5'→3' exonuclease activity are preferred. Examples of such enzymes include Sequenase, recombinant Taq DNA polymerase, and similar enzymes whose exonuclease activity has been eliminated. Even if enzymes have an exonuclease activity, there is no problem when the enzymes are reacted under conditions under which the exonuclease activity is weak. Therefore, T4 DNA polymerase, DNA polymerase I, natural Taq DNA polymerase, and similar enzymes may be used. Furthermore, instead of an oligonucleotide with an unphosphorylated 5' end, a phosphorylated oligonucleotide may be used. In this case, treatment with a DNA polymerase can be omitted. However, since the oligonucleotides may be ligated to each other, resulting in competitive inhibition of subsequent PCR reactions, procedures for removing low molecular weight DNA, such as gel filtration, spin column, ethanol precipitation, isopropanol precipitation, and polyethylene glycol precipitation, are preferably performed. In step (5) described later, a polymerase chain reaction occurs by binding of the second oligonucleotide or the like to the sequence of the first oligonucleotide. Therefore, the first oligonucleotide preferably has a sufficient length that enables hybridization with the whole second oligonucleotide. However, when treatment with a DNA polymerase is performed, the first oligonucleotide may be a short oligonucleotide that can be hybridized with only a part of the second oligonucleotide. In this case, since a complementary strand of the second oligonucleotide is synthesized during the process in which a DNA polymerase repairs a gap between the first oligonucleotide and the first enzyme cleavage end, a portion corresponding to the first oligonucleotide has a sequence hybridizable with the whole second oligonucleotide as a result.

Step (3) is digesting the DNA fragment obtained in step (2) with the second restriction enzyme. The second restriction enzyme is not particularly limited, and enzymes that produce a short cleavage fragment are preferred. Preferred examples include enzymes recognizing four nucleotides such as *Taq*I. Furthermore, when the methylation state of a genomic DNA is analyzed, a non-methylation-sensitive restriction enzyme is preferably used as the second restriction enzyme. The second restriction enzyme may also be an enzyme that produces any kind of end, as with the first restriction enzyme.

Step (4) is allowing the double-stranded DNA fragment produced in step (3) to coexist with the third oligonucleotide with an unphosphorylated 5' end and the fourth oligonucleotide with an unphosphorylated 5' end having a sequence complementary to the third oligonucleotide in the presence of a ligase, threreby adding a double-stranded DNA fragment consisting of the third oligonucleotide and the fourth oligonucleotide to the second restriction enzyme cleavage end of the double-stranded DNA fragment produced in step (3) to obtain a DNA fragment, in which the 3' end of the fourth oligonucleotide is ligated to the restriction enzyme cleavage end of the double-stranded DNA fragment produced in step (3), but the 5' end of the third oligonucleotide is not ligated to the restriction enzyme cleavage end of the double-stranded DNA fragment produced in step (3). Ligases similar to the ligases explained in step (2) can be used. Furthermore, oligonucleotides similar to the first oligonucleotide and the second oligonucleotide explained in step (2) can also be used as the third oligonucleotide and the fourth oligonucleotide. However, when the sequences are identical or similar, such a problem may arise that the primer to be hybridized with the first oligonucleotide in step (5) may be hybridized with the third oligonucleotide. Therefore, oligonucleotide sequences that are not similar should be used. Furthermore, since the third oligonucleotide-dependent PCR reaction may produce nonspecific reaction products, it is preferable that oligonucleotides should not be hybridized with the fourth oligonucleotide or the like under PCR reaction conditions.

Step (5) is heating the double-stranded DNA fragment obtained in step (4) thereby dissociating the double-stranded DNA fragment, and then performing a polymerase chain reaction using an oligonucleotide hybridizable with the first oligonucleotide and oligonucleotide hybridizable with the third oligonucleotide as primers to selectively amplify a DNA fragment having the cleavage end of the first restriction enzyme and the cleavage end of the second restriction enzyme. The temperature at the time of heating can be similar to temperature in usual hot start PCR, and appropriate temperature is approx. 90 to 95°C. The polymerase chain reaction conditions (temperature, number of cycles, polymerase used, etc.) can also be similar to those in usual hot start PCR. Examples of the oligonucleotide hybridizable with the first oligonucleotide used as a primer include the second oligonucleotide, an oligonucleotide obtained by removing one or several nucleotides of the second oligonucleotide, an oligonucleotide obtained by adding another sequence to the above-mentioned oligonucleotides to an extent that the function as a primer should not be affected, and so forth. Furthermore, examples of the oligonucleotide hybridizable with the third oligonucleotide include the fourth oligonucleotide, an oligonucleotide obtained by removing one or several nucleotides of the fourth oligonucleotide, an oligonucleotide obtained by adding another sequence to the above-mentioned oligonucleotides to an extent that the function as a primer should not be affected, and so forth. The length of these oligonucleotides used as primers is not particularly limited and is preferably 17 to 50 nucleotides.

The method for amplifying a DNA fragment of the present invention can be used to measure the methylation degree of a genomic DNA. Specifically, using a genomic DNA as a sample double-stranded DNA, a methylation-sensitive restriction enzyme as the first restriction enzyme, and a non-methylation-sensitive restriction enzyme as the second restriction enzyme, the position of a DNA fragment amplified on the genomic DNA is identified, the amplification amount of a DNA fragment including the cleavage site is determined for each methylation-sensitive restriction enzyme cleavage site, and the methylation degree of the methylation-sensitive restriction enzyme cleavage site can be measured. For example, when a small amount of DNA fragments including a certain methylation-sensitive restriction enzyme cleavage site are amplified, the cleavage site can be determined to be in a hypermethylated state. On the contrary, when a large amount of DNA fragments are amplified, the cleavage site can be determined to be in a hypomethylated state. The method for identifying the position of the amplified DNA fragment on the genome is not particularly limited, and a genome tiling array or the like can be used.

The method for amplifying a DNA fragment of the present invention can be used for analyses of single nucleotide polymorphism, detection or analyses of recombination of DNA including transposition, deletion, translocation, and the like, and point or region mutation, and the like in addition to measurement of the methylation degree of the genomic DNA.

### Examples

Hereafter, the present invention will be explained more specifically with the following examples. Reference Example In silico genomic sequence analysis

Restriction enzyme sites of a mouse genome sequence (mm6) obtained from the UCSC Genome Browser Database (http://genome.ucsc.edu/) were analyzed with a sequence analysis software (EMBOSS Package), and detailed analyses were further performed by using the obtained results in software programs such as Excel, File Maker, Perl, R, and Python.

For example, 6012 *Not*I recognition sites exist in the mouse genome sequence (of the genomic sequences, ChrM, a random sequence of chromosome, is not included). In a histogram showing a distribution of lengths of these fragments (Figure 3A illustrates an analysis example of chromosome 17), most of the fragments have a length exceeding 8 kb, and the average chain length exceeds 300 kb. The lengths of fragments efficiently amplified by PCR are 3 kb and shorter. Furthermore, the lengths of fragments efficiently recovered during the process of DNA purification are 50 bp or longer. Therefore, even if the fragment cleaved with *Not*I is to be amplified by LM-PCR, most of the fragments are not amplified.

On the other hand, in a histogram similarly showing a distribution of chain lengths of fragments cleaved with *Not*I and *Taq*I (Figure 3B shows an analysis example of chromosome 1), the average chain length of fragments including the *Not*I site at an end is approx. 1.25 kb, and most fragments (88%) are 3 kb or shorter. In contrast, approx. 15% are 50 nucleotides or shorter in length. Therefore, approx. 70% of fragments are distributed in the lengths that enable PCR amplification. Fragments are on both sides of a single *Not*I cleavage site, and both the fragments have 127 *Not*I cleavage sites which cannot be analyzed (50 nucleotides or shorter in length or larger than 3 kb). Therefore, fragments cleaved with *Not*I and *Taq*I are amplified by PCR, and the percentage of *Not*I sites that cannot be determined by the occurrence of amplification of a fragment is approx. 2%.

Meanwhile, approx. 200 times more *Taq*I recognition sites than *Not*I sites exist, and the average chain length of fragments are approx. 1.8 kb. Therefore, most fragments cleaved with *Taq*I are in the fragment length range in which fragments are very likely to be amplified by PCR. It is found that, if a fragment having only the *Taq*I cleavage end is similarly amplified as with the fragment having the *Not*I cleavage end, most amplified fragments are fragments having only the *Taq*I cleavage end at an end thereof.

The restriction sites of *HpyCH4*IV were similarly analyzed. The mouse genome has approx. 3.5 million *HpyCH4*IV recognition sites (of the genomic sequences, ChrM, a random sequence of chromosome, is not included). As a representative, an example of an analysis using the sequence of mouse chromosome 17 will be described. Chromosome 17 has 64,250 *HpyCH4*IV recognition sites. In a histogram showing a distribution of fragment lengths (Figure 4A shows an analysis example), the average chain length is approx. 1.4 kb, and approx. 90% of fragments are 3 kb or shorter in length. Therefore, if it is assumed that all the *HpyCH4*IV restriction sites are in a non-methylated state, all fragments are in the chain length range that enables PCR amplification. However, when the normal mouse liver genome is cleaved with *HpyCH4*IV, and distributions of cleaved fragments are examined by electrophoresis, it is found that most fragments have a length exceeding 10 kb (Figure 5). Therefore, most of fragments cleaved with *HpyCH4*IV alone are not amplified by LM-PCR.

As in the case of *Not*I, in a histogram showing a distribution of chain lengths of fragments cleaved with *HpyCH4*IV and *Taq*I (Figure 4B shows an analysis example of chromosome 17), the average chain length of fragments including the *HpyCH4*IV site at an end thereof is approx. 0.79 kb, and most fragments (97%) are 3 kb or shorter in length. On the other hand, the percentage of fragments of 50 nucleotides or shorter is approx. 6% after cleavage with *HpyCH4*IV and approx. 8% after cleavage with *HpyCH4*IV and TaqI, which shows virtually no increase. Therefore, by using TaqI in combination, most fragments digested with *HpyCH4*IV have lengths that enable PCR amplification.

### Example 1 Preparation of genomic DNA

The genomic DNA of a target cell was prepared as follows by a known method (Molecular Cloning; A Laboratory Manual). Each frozen cell sample (approx. 1 or 2 × 10⁶ cells) was dissolved in 100 µl of lysis buffer [containing 5 mM EDTA, 100 mM Tris-HCl [pH 8.5], 0.2% SDS, proteinase K (200 µg/ml)], and the mixture was incubated at 55°C for 30 min. The mixture was extracted twice with phenol/chloroform/isoamyl alcohol (50:49:1), and the genomic DNA was precipitated by ethanol precipitation and dissolved in 20 µl of TE (10 mM Tris-HCl, 1 mM EDTA [pH 7.6]).

### Example 2 Selective amplification of DNA fragment having NotI cleavage end and TaqI cleavage end

A methylation-sensitive restriction enzyme *Not*I (Takara) and a non-methylation-sensitive restriction enzyme *Pvu*II (Takara) were added to 5 µg of the genomic DNA diluted in a restriction enzyme buffer and reacted overnight at 37°C to digest the genomic DNA. The mixture was extracted with phenol/chloroform (1:1) to recover an aqueous layer containing DNA, followed by the addition of 1/10 of the volume of 3 M sodium acetate solution (pH 5.2), and then the genomic DNA was precipitated by ethanol precipitation. The recovered DNA was dissolved in 5 µl of TE solution, and 1/10 of the volume thereof was subjected to agarose electrophoresis to confirm the cleavage of DNA fragments. 1 pmol of adaptors (R24 and Rggcc12) were added to 1/10 of the volume of DNA, followed by the addition of a T4 ligase buffer, and then the mixture was heated at 65°C for 5 min and gradually cooled to 16°C over approx. 1 h. T4 DNA ligase (NEB) was added here and reacted overnight at 16°C to ligate the genomic DNA cleaved with the restriction enzyme and the adaptors. Of the adaptors used, R24 has a sequence complementary to Rggcc12, and Rggcc12 has a sequence complementary to the overhang portion produced by *Not*I. After the overnight reaction, a dNTP mixture solution and a Klenow fragment (Takara) were added, and a reaction was further performed at 16°C for 30 min. The reaction mixture was heated at 70°C for 10 min to inactivate the DNA ligase and the Klenow fragment. A sodium chloride solution was added here at a final concentration of 100 mM, followed by the addition of restriction enzyme *Taq*I, and the mixture was reacted at 65°C for 3 h to completely cleave the DNA. The mixture was extracted with phenol/chloroform (1:1) to recover an aqueous layer containing DNA, unreacted adaptors were removed using MicroSpin Column (GE Healthcare), followed by the addition of 1/10 of the volume of 3 M sodium acetate solution (pH 5.2), and DNA was precipitated by ethanol precipitation. DNA was dissolved in an appropriate amount of sterilized water, followed by the addition of 100 pmol of adaptors (addition of equimolar amounts of N24 and Ncg10) and a T4 ligase buffer, and then the mixture was heated at 65°C for 5 min and then gradually cooled to 16°C over approx. 1 h. T4 DNA ligase (NEB) was added here, and the mixture was reacted overnight at 16°C to ligate the DNA cleaved with restriction enzyme *Taq*I and the adaptors. Of the adaptors used, N24 has a sequence complementary to *Ncg*10, and *Ncg*10 has a sequence complementary to the overhang portion produced by *Taq*I. After completion of the reaction, DNA fragments were purified using a DNA purification kit (Wizard SV Gel and PCR Clean-Up System, Promega) to remove unreacted adaptors and the like. A part of the recovered DNA was added to a modified heat-resistant DNA polymerase reaction mixture containing primers N18 and R182 and treated in a reaction mixture containing dNTP at 95°C for 7 min, ant then a reaction was performed 20 cycles consisting of heating cycles at 94°C for 30 s, 62°C for 30 s, and 72°C for 2 min. Of the primers used, N18 has a sequence obtained by removing one to several nucleotides from an end of N24, and R182 has a sequence obtained by removing one to several nucleotides from an end of R24. As controls, reaction mixtures containing N18 alone or R182 alone as a primer were prepared and similarly reacted. In the controls, when DNA is cleaved with *Taq*I alone, fragments can be amplified, and all genome sequences having a methylated *Not*I cleavage site are amplified.

According to the principle of usual LM-PCR, fragments having only the *Not*I cleavage end at both ends are amplified in the PCR reaction mixture containing R182 alone, fragments having only the *Taq*I cleavage end at both ends are amplified in the PCR reaction mixture containing N18 alone, and fragments having *Not*I and *Taq*I cleavage ends are amplified in the reaction mixture containing N18 and R182 in addition to the above-mentioned two kinds of fragments.

Before amplification, fragments having only the *Taq*I cleavage end at both ends are most abundant. Even if it is assumed that all *Not*I sites in the genome are non-methylated, approx. 250 times more fragments having only the *Taq*I cleavage end at both ends exist than fragments having *Not*I-*Taq*I ends. Therefore, when usual LM-PCR is performed, fragments amplified with N18 will be the most abundant. As shown in Figure 6, however, when N18 alone was added as a primer, amplification products were hardly detected. On the other hand, when N18 and R182 were added, a large amount of amplification products were detected. Furthermore, since only a small amount of amplification products were detected when R182 alone was added, it is found that many of fragments amplified in the reaction mixture containing N18 and R182 are fragments having the *Taq*I cleavage end at one end and the *Not*I cleavage end at the other end.

After completion of the reaction, the PCR products in the reaction mixture containing N18 and R182 were purified using QIAquick Mini Elute Kit. Furthermore, for comparison, usual LM-PCR was performed using N18 as a primer, and the PCR products were purified using QIAquick Mini Elute Kit.

A total of 9 µg each of the above-mentioned two kinds of purified PCR products were sent to NimbleGen, and the products of usual LM-PCR using N18 as a primer were labeled with Cy5, the products amplified by the method of the present invention using both N18 and R182 as primers were labeled with Cy3, and these products were hybridized with a tiling array containing sequences surrounding the *Not*I sequence in a competitive hybridization system to obtain signals. Here, the sequences surrounding *Not*I means a region including 2.5 kb upstream and 2.5 kb downstream of *Not*I based on the information obtained by extracting the *Not*I restriction site from a mouse genome sequence (mm7) obtained from the UCSC Genome Browser Database (http://genome.ucsc.edu/) using EMBOSS (Trends in Genetics 16, 276-277). A probe sequence was selected so that one 50 nucleotide-long probe should be included per 100 base pairs in these regions. The probe corresponding to the repeat sequence (Repeat Mask) was removed.

### Example 3 Analysis by histogram of hybridization signals

Of the analysis files provided by NimbleGen, data with normalized signals using the q-spline function were analyzed. The obtained results were further analyzed in detail using software programs such as Excel, File Maker, Perl, R, and Python.

It was assumed that all probes on the microarray were completely cleaved, and probes that were longer than 50 bp and 5 kb and shorter, were classified into probes existing in a *Not*I*-Taq*I fragment or a *Not*I-*Not*I fragment and other probes (mainly probes existing in a *Taq*I*-Taq*I fragment) (hereinafter, the former is referred to as *"Not*I*-Taq*I group" and the latter as *"Taq*I*-Taq*I group").

Logarithmic values of the fluorescence intensity ratio of Cy3 and Cy5 of the probes belonging to the *Not*I-*Taq*I group (Cy5/Cy3) were obtained and plotted in a histogram (Figure 7A). As shown in Figure 7A, the histogram did not show a normal distribution, but a distribution biased to the negative side. That is, the groups with lower Cy5/Cy3 values were significantly amplified. Such a distribution suggests that the amount of the amplification products by the method of the present invention was different from that of the amplification products of usual LM-PCR, and that the *Not*I*-Taq*I fragments had been amplified by the method of the present invention.

Meanwhile, a histogram was similarly prepared for the probes belonging to the *Taq*I*-Taq*I group (Figure 7B). As shown in Figure 7B, the histogram showed a normal distribution. Thus, the ratio of the amplification products by the method of the present invention and the amplification products of usual LM-PCR showed a normal distribution in the *Taq-Taq* group, but a normal distribution was not observed in the *Not*I*-Taq*I group. This means that *Not*I*-Taq*I fragments and *Not*I-*Not*I fragments had been selectively amplified by the method of the present invention.

### Example 4 Analysis of hybridization signal by IGB

The signals for hybridization of amplification products by the method of the present invention and probes on the microarray were expressed using Integrated Genome Browser (IGB, Affymetrix, Inc.).

As illustrated in Figure 8-1A, most microarray signals originated from the probes belonging to the *Not*I-*Taq*I group (lower column in Figure 8-1A), and virtually no signals originating from probes belonging to the *TaqI-Taq*I group were detected (middle column in Figure 8-1A). Furthermore, microarray signal detected sites were corresponding to the *Not*I cleavage sites (Figure 8-1B). These results suggest that only *Not*I*-Taq*I fragments and *Not*I-*Not*I fragments are selectively amplified by the amplification method of the present invention.

As shown in Figure 8-2, no signal was detected at methylated *Not*I cleavage sites (Figure 8-2A), and signals were detected at non-methylated *Not*I cleavage sites (rightmost *Not*I cleavage site in Figure 8-2B). This suggests that the amplification method of the present invention is effective for analyses of methylation states. Since no signal was detected at two *Not*I cleavage sites on the left in Figure 8-2B, they are considered to be methylated.

### Example 5 Selective amplification of DNA fragments having HpyCH4IV cleavage end and TaqI cleavage end

A methylation-sensitive restriction enzyme *HpyCH4*IV (NEB) was added to 5 µg of a genomic DNA diluted in a restriction enzyme buffer and reacted overnight at 37°C to digest the genomic DNA. The mixture was extracted with phenol/chloroform (1:1) to recover an aqueous layer containing DNA, followed by the addition of 1/10 of the volume of 3 M sodium acetate solution (pH 5.2), and then the genomic DNA was precipitated by ethanol precipitation. The recovered DNA was dissolved in 5 µl of TE solution, and 1/10 of the volume of the mixture was subjected to agarose electrophoresis to confirm the cleavage of DNA fragments. 100 pmol of adaptors (R24 and R10) were added to 1/10 of the volume of DNA, followed by the addition of a T4 ligase buffer, and the mixture was heated at 65°C for 5 min and then gradually cooled to 16°C over approx. 1 h. T4 DNA ligase (NEB) was added here, and this mixture was reacted overnight at 37°C to ligate the genomic DNA cleaved with the restriction enzyme and the adaptors. Of the adaptors used, R10 has a sequence complementary to the *HpyCH4*IV cleavage end. After overnight reaction, a dNTP mixture solution and a Klenow fragment (Takara) were added, and a reaction was further performed at 16°C for 30 min. The reaction mixture was heated at 70°C for 10 min to inactivate the DNA ligase and the Klenow fragment. A sodium chloride solution was added here at a final concentration of 100 mM, followed by the addition of restriction enzyme *Taq*I, and the mixture was reacted at 65°C for 3 h to completely cleave the DNA. The mixture was extracted with phenol/chloroform (1:1) to recover an aqueous layer containing DNA, followed by the addition of 1/10 of the volume of 3 M sodium acetate solution (pH 5.2), and DNA was precipitated by ethanol precipitation. DNA was dissolved in an appropriate amount of sterilized water, followed by the addition of 100 pmol of adaptors (addition of equimolar amounts of N24 and Ncg10), followed by the addition of a T4 ligase buffer, and the mixture was heated at 65°C for 5 min and then gradually cooled to 16°C over approx. 1 h. T4 DNA ligase (NEB) was added here, and the mixture was reacted overnight at 16°C to ligate the DNA cleaved with restriction enzyme *Taq*I and the adaptors. After completion of the reaction, DNA fragments were purified using a DNA purification kit (Wizard SV Gel and PCR Clean-Up System, Promega) to remove unreacted adaptors and the like. A part of the recovered DNA was added to a modified heat-resistant DNA polymerase reaction mixture containing primers N18 and R182 and treated in a reaction mixture containing dNTP and dUTP at 95°C for 7 min, and a reaction was performed 20 cycles consisting of thermal cycles at 94°C for 30 s, 62°C for 30 s, and 72°C for 2 min. As controls, reaction mixtures containing primer N18 alone or R182 alone were prepared and subjected to similar reactions. As shown in Figure 9, fragments amplified by N18 alone that have only the *Taq*I end showed the lowest amplification efficiency. On the other hand, PCR products were abundant in the reaction mixture containing N18 and R182 and in the reaction mixture containing R182 alone. It is found that many of fragments amplified in the reaction mixture containing N18 and R182 are fragments having the *Taq*I cleavage end at one end and the *HpyCH4*IV cleavage end at the other end and fragments having the *HpyCH4*VI end at both ends. After completion of the reaction, PCR products were purified using QIA Quick Mini Elute Kit.

A total of 9 µg of PCR products amplified using primers N18 and R182 were fragmented using Gene Chip WT Double-Stranded DNA Terminal Labeling Kit (Affymetrix) according to a method recommended by the manufacturer and labeled with biotin. According to a method recommended by the manufacturer, the products were hybridized with a mouse promoter-tiling array to obtain hybridization signals. Signals were analyzed by GCOS to obtain files (.CEL) in which results were compiled as data.

### Example 6 Analysis of hybridization signals

For analysis of the .CEL file, R and MAT were used (Proc Natl Acad Sci USA. 103: 12457-12462). Using the same liver DNA, preparation of fragments and hybridization with the microarray described in Example 5 were performed twice. Correlations of the signal intensity on the microarray were plotted to obtain the correlation coefficient (Figure 10). The correlation coefficient was very high, with R² = 0.944, which confirmed high reproducibility.

By performing analyses using MAT, the hybridization signal values are converted to MATscores, to which effect of probe sequences on signal intensity and redundancy of a probe is added, and can be expressed with IGB. Results of MAT scores of regions of which methylation state had already been found by 1GB are shown in Figure 11A. As shown in Figure 11A, MATscores were high in hypomethylated sites and low in hypermethylated sites. This suggests that the amplification method of the present invention is effective for analyses of methylation status.

Furthermore, the region with a high MAT score was centered around the *HpyCH4*IV cleavage site and overlapped a region surrounded by the *HpyCH4*IV cleavage site and the *Taq*I cleavage site. Therefore, it was confirmed that *HpyCH4*IV-TaqI fragments had been selectively amplified by the amplification method of the present invention.

When distributions of MATscores were compared between the probes existing in a fragment including the *HpyCH4*IV cleavage site and the other probes, the probes existing in a fragment including the *HpyCH4*IV cleavage site showed a distribution of high scores as shown in Figure 12, and selective amplification of *HpyCH4*IV-*Taq*I fragments was confirmed.

The sequences of the adaptors and primers used in this example are listed below.
N24: AGGCAACTGTGCTATCCGAGGGAA (SEQ ID NO: 1)
Rggcc12: GGCCAGCGGTGA (SEQ ID NO: 2)
N18: GGCAACTGTGCTATCCGA (SEQ ID NO: 3)
Ncg10: CGTTCCCTCG (SEQ ID NO: 4)
R24: GCACTCTCCAGCCTCTCACCGCT (SEQ ID NO: 5)
R10: CGAGCGGTGA (SEQ ID NO: 6)
R182: GCACTCTCCAGCCTCTCA (SEQ ID NO: 7)

This specification encompasses contents described in the specification and/or drawings of Japanese Patent Application No. 2007-056839, based on which the conventional priority of the present application was claimed. Furthermore, various publications, patents, and patent applications referred to throughout the present invention in their entireties are hereby incorporated by reference into this specification.

## Claims

1. A method for amplifying a DNA fragment,
**characterized by** comprising the following steps (1) to (5):
(1) digesting a sample double-stranded DNA with a first restriction enzyme;
(2) adding a double-stranded DNA fragment consisting of a first oligonucleotide and a second oligonucleotide having a sequence complementary to the first oligonucleotide to a restriction enzyme cleavage end of the double-stranded DNA fragment produced in step (1) to obtain a DNA fragment, in which the 5' end of the first oligonucleotide and the 3' end of the second oligonucleotide are ligated to the restriction enzyme cleavage end of the double-stranded DNA fragment produced in step (1);
(3) digesting the DNA fragment obtained in step (2) with a second restriction enzyme;
(4) allowing the double-stranded DNA fragment produced in step (3) to coexist with a third oligonucleotide with an unphosphorylated 5' end and a fourth oligonucleotide with an unphosphorylated 5' end that has a sequence complementary to the third oligonucleotide in the presence of a ligase, threreby adding a double-stranded DNA fragment consisting of the third oligonucleotide and the fourth oligonucleotide to the second restriction enzyme cleavage end of the double-stranded DNA fragment produced in step (3) to obtain a DNA fragment, in which the 3' end of the fourth oligonucleotide is ligated to the restriction enzyme cleavage end of the double-stranded DNA fragment produced in step (3), but the 5' end of the third oligonucleotide is not ligated to the restriction enzyme cleavage end of the double-stranded DNA fragment produced in step (3); and
(5) heating the double-stranded DNA fragment obtained in step (4) thereby dissociating the double-stranded DNA fragment, and then performing a polymerase chain reaction using an oligonucleotide hybridizable with the first oligonucleotide and an oligonucleotide hybridizable with the third oligonucleotide as primers to selectively amplify a DNA fragment having the cleavage end of the first restriction enzyme and the cleavage end of the second restriction enzyme.

2. The method for amplifying a DNA fragment according to claim 1, **characterized in that** the step (2) is allowing the double-stranded DNA fragment produced in step (1) to coexist with the first oligonucleotide with an unphosphorylated 5' end and the second oligonucleotide with an unphosphorylated 5' end in the presence of a ligase, threreby adding a double-stranded DNA fragment consisting of the first oligonucleotide and the second oligonucleotide to the restriction enzyme cleavage end of the double-stranded DNA fragment produced in step (1) to obtain a DNA fragment, in which the 3' end of the second oligonucleotide is ligated to the restriction enzyme cleavage end of the double-stranded DNA fragment produced in step (1), but the 5' end of the first oligonucleotide is not ligated to the restriction enzyme cleavage end of the double-stranded DNA fragment produced in step (1), and then allowing a DNA polymerase to act to ligate the double-stranded DNA fragment produced in step (1) and the 5' end of the first oligonucleotide.

3. The method for amplifying a DNA fragment according to claim 1, **characterized in that** the step (2) is allowing the double-stranded DNA fragment produced in step (1) to coexist with the first oligonucleotide with a phosphorylated 5' end and the second oligonucleotide with a phosphorylated 5' end in the presence of a ligase, threreby adding a double-stranded DNA fragment consisting of the first oligonucleotide and the second oligonucleotide to the restriction enzyme cleavage end of the double-stranded DNA fragment produced in step (1) to obtain a DNA fragment, in which the 5' end of the first oligonucleotide and the 3' end of the second oligonucleotide are ligated to the restriction enzyme cleavage end of the double-stranded DNA fragment produced in step (1), and then removing the first oligonucleotide, the second oligonucleotide, and the DNA fragment produced by ligation.

4. The method for amplifying a DNA fragment according to any one of claims 1 to 3, **characterized in that** the first restriction enzyme and the second restriction enzyme are restriction enzymes which leave a 5' overhang, and the first oligonucleotide and the third oligonucleotide are oligonucleotides having a sequences complementary to the 5' overhangs generated by the first restriction enzyme and the second restriction enzyme, respectively.

5. A method for measuring the methylation degree of a genomic DNA by allowing a methylation-sensitive restriction enzyme to act on the genomic DNA, amplifying the resulting DNA fragment, and determining the methylation degree of a cleavage site of the methylation-sensitive restriction enzyme based on the amplification degree, **characterized by** comprising the following steps (1) to (6):
(1) digesting a genomic DNA with a methylation-sensitive restriction enzyme;
(2) adding a double-stranded DNA fragment consisting of a first oligonucleotide and a second oligonucleotide having a sequence complementary to the first oligonucleotide to a restriction enzyme cleavage end of a DNA fragment produced in step (1) to obtain a DNA fragment, in which the 5' end of the first oligonucleotide and the 3' end of the second oligonucleotide are ligated to the restriction enzyme cleavage end of the DNA fragment produced in step (1);
(3) digesting the DNA fragment obtained in step (2) with a non-methylation-sensitive restriction enzyme;
(4) allowing the double-stranded DNA fragment produced in step (3) to coexist with a third oligonucleotide with an unphosphorylated 5' end and a fourth oligonucleotide with an unphosphorylated 5' end that has a sequence complementary to the third oligonucleotide in the presence of a ligase, threreby adding a double-stranded DNA fragment consisting of the third oligonucleotide and the fourth oligonucleotide to a non-methylation-sensitive restriction enzyme cleavage end of the double-stranded DNA fragment produced in step (3) to obtain a DNA fragment, in which the 3' end of the fourth oligonucleotide is ligated to the restriction enzyme cleavage end of the double-stranded DNA fragment produced in step (3), but the 5' end of the third oligonucleotide is not ligated to the restriction enzyme cleavage end of the double-stranded DNA fragment produced in step (3);
(5) heating the double-stranded DNA fragment obtained in step (4) thereby dissociating the double-stranded DNA fragment, and then performing a polymerase chain reaction using an oligonucleotide hybridizable with the first oligonucleotide and an oligonucleotide hybridizable with the third oligonucleotide as primers to selectively amplify a DNA fragment having a cleavage end of a methylation-sensitive restriction enzyme and a cleavage end of a non-methylation-sensitive restriction enzyme; and
(6) identifying the position of the amplified DNA fragment on the genomic DNA and determining the amplification amount of a DNA fragment including a cleavage site for each methylation-sensitive restriction enzyme.

6. The method for measuring the methylation degree of genomic DNA according to claim 5, **characterized in that** the step (2) is allowing the double-stranded DNA fragment produced in step (1) to coexist with a first oligonucleotide with an unphosphorylated 5' end and a second oligonucleotide with an unphosphorylated 5' end in the presence of a ligase, and adding a double-stranded DNA fragment consisting of the third oligonucleotide and the fourth oligonucleotide to a restriction enzyme cleavage end of the double-stranded DNA fragment produced in step (1) to obtain a DNA fragment, in which the 3' end of the second oligonucleotide is ligated to the restriction enzyme cleavage end of the double-stranded DNA fragment produced in step (1), but the 5' end of the first oligonucleotide is not ligated to the restriction enzyme cleavage end of the double-stranded DNA fragment produced in step (1), and then allowing a DNA polymerase to act to ligate the double-stranded DNA fragment produced in step (1) and the 5' end of the first oligonucleotide.

7. The method for measuring the methylation degree of genomic DNA according to claim 5, **characterized in that** the step (2) is allowing the double-stranded DNA fragment produced in step (1) to coexist with a first oligonucleotide with a phosphorylated 5' end and a second oligonucleotide with a phosphorylated 5' end in the presence of a ligase, threreby adding a double-stranded DNA fragment consisting of the first oligonucleotide and the second oligonucleotide to a restriction enzyme cleavage end of the double-stranded DNA fragment produced in step (1) to obtain a DNA fragment, in which the 5' end of the first oligonucleotide and the 3' end of the second oligonucleotide are ligated to the restriction enzyme cleavage end of the double-stranded DNA fragment produced in step (1), and removing the first oligonucleotide, the second oligonucleotide, and the DNA fragment produced by ligation.

8. The method for measuring the methylation degree of genomic DNA according to any one of claims 5 to 7,
**characterized in that** the methylation-sensitive restriction enzyme and the non-methylation-sensitive restriction enzyme are restriction enzymes that leave a 5' overhang, and the first oligonucleotide and the third oligonucleotide are oligonucleotides having sequences complementary to the 5' overhangs generated by the methylation-sensitive restriction enzyme and the non-methylation-sensitive restriction enzyme, respectively.
